# EUROPEAN PATENT SPECIFICATION

(11) **EP 0 542 996 B1**
(45) Date of publication and mention of the grant of the patent: **11.08.1999**
(21) Application number: 92913831.1
(22) Date of filing: 27.05.1992
(51) Int. Cl.: C12P 19/04, C12P 19/00, C07H 13/02, C07H 15/04, C08B 37/00

(54) **SUGAR-BASED POLYMERS**
POLYMERE AUF ZUCKERBASIS
POLYMERES A BASE DE SUCRE

(30) Priority: 28.05.1991 US 706929
(43) Date of publication of application: 26.05.1993
(73) Proprietor: UNIVERSITY OF IOWA RESEARCH FOUNDATION, Oakdale, IA 52319 (US)
(72) Inventor: DORDICK, Jonathan, S., Iowa City, IA 52240 (US); RETHWISCH, David, G., Iowa City, IA 52245 (US); PATIL, Damodar, R., East Grand Forks, MN 56721 (US)
(74) Representative: Baldock, Sharon Claire
(86) International application number: US9204517
(87) International publication number: WO9221765

(56) References cited:
- WO-A-91/17255
- DD-A- 148 058
- US-A- 3 265 641
- Derwent Publications Ltd., London, GB; AN 83-62939K & JP-A-58 087174 (NIPPON URETHANE) 24 May 1983
- Derwent Publications Ltd., London, GB; AN 92-223547 & JP-A-4 149270 (DAINIPPON INK) 22 May 1992
- Biotechnology and Bioengineering, Volume 37, issued March 1991, D. R. PATIL et al., "Enzymatic Synthesis of a Sucrose-Containing. Linaer Polyester in Nearly Anhydrous Organic Media" pages 639-646 DD,A, 148,058 (VEB Synthesewerk Schwarzheide) 06 May 1981 (with English translation). see entire document.

## Description

The present invention relates to the field of sugar-based polymers and, more particularly, to a method of making a poly(sugar-acrylate) and the resulting poly(sugar-acrylate).

The present invention is directed to novel polymers which incorporate an abundant, relatively inexpensive and recyclable resource, sugar. The present invention is also directed to novel methods of making these polymers. It is contemplated that the sugar-based polymers of the present invention will find significant use in diaper liners as well as in other absorbent materials, packaging materials, drug delivery polymers, and in a variety of other commercial applications.

Biotechnology and Bioengineering, 37, 1991, 639-46 discloses a sucrose containing linear polymer and a method for making this polyester where the sucrose moiety is incorporated into the polymer backbone itself.

The sugar-based polymers of the present invention are manufactured pursuant to a combination of enzymatic and chemical synthesis (i.e. chemoenzymatic synthesis). In particular, hydrolytic enzymes are used to regioselectively acylate sugar molecules with organic acid derivatives. The acylated sugar intermediates are then polymerized via chemical methods.

The present invention contemplates the utilization of mono-, di-, tri- and oligosaccharides. Preferred sugars are glucose, mannose, fructose (monosaccharides); sucrose, lactose, maltose, trehalose (disaccharides); and raffinose (a trisaccharide). More preferred sugars for use in the present invention include sucrose, fructose, raffinose, lactose, maltose and trehalose. Even more preferred sugars, however, are fructose, sucrose and raffinose. The most preferred sugar is sucrose.

### PREPARATION OF POLY (SUGAR ACRYLATES)

According to a first aspect of the present, invention, there is provided a chemoenzymatic method for making a poly (sugar acrylate), comprising the steps of:
(a) in the presence of a hydrolytic enzyme reacting an acylating compound with a non-reducing sugar in a substantially non-aqueous organic solvent to form the acryloyl ester of the sugar; the acylating compound having the general formula: 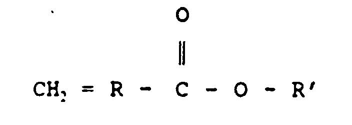 wherein R is selected from the group consisting of alkanes, alkenes, branched alkanes, substituted alkenes, substituted aliphatic moieties, aromatic moieties, substituted aromatic moieties and mixtures thereof, and R' is a moiety that is replaced by the non-reducing sugar,
(b) heating the acryloyl ester of the sugar so that it autopolymerizes to form a poly (sugar acrylate); and
(c) isolating the resulting poly (sugar acrylate).

Oligosaccharides that are non-reducing sugars are contemplated for use in the present embodiment. Any reducing monosaccharide, however, can be converted to a non-reducing sugar by either alkylating or halogenating the 1-position. Preferably, the sugar is selected from the group consisting of α- and β-alkylglucosides, α- and β- haloglucosides, α- and β-alkylgalactosides, α- and β- halogalactosides, α- and β- alkylmannosides, α- and β- halomannosides, sucrose, fructose, mannose trehalose and raffinose. Preferred sugars are α- and β- methylglucoside, α- and β- methyl galactoside, α- and β- methylmannoside, sucrose, fructose, mannose, trehalose and raffinose. More preferred sugars are sucrose, fructose and raffinose. The most preferred sugar is sucrose.

Specifically, a sugar is first acylated with an acylating compound having the general formula: 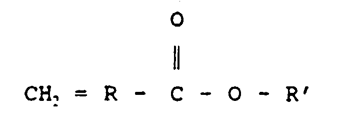 wherein R is selected from the group consisting of alkanes, alkenes, branched alkanes, substituted alkenes, substituted aliphatic moieties, aromatic moieties, substituted aromatic moieties and mixtures thereof, and R' is a moiety that is replaced by the non-reducing sugar.

It is presently believed that the sugar is acylated with the acylating compound pursuant to a nucleophilic mechanism. As presently understood, the sugar molecules replace R' by an enzyme-organic acid derivative intermediate via a nucleophilic mechanism. When R' is a poorer nucleophile than the sugar there will be little competition between these groups and the sugar molecules thus resulting in a greater yield of poly sugar acrylates. Accordingly, R' is preferably a good leaving group. By a leaving group it is meant that R' may be any group that is replaced by sugar in the presence of a hydrolytic enzyme with a poorer nucleophile than the sugar.

A preferred acylating compound is vinyl acrylate.

According to the present aspect of the invention the sugar is acylated by mixing the sugar and acylating compound in a substantially non-aqueous, organic solvent.

If the solubility of the sugar and organic acid derivative is substantially less than about 10 mmol/l liter solvent, the manufacture of the polymers of the present invention may not be economically desirable. Sugars are reasonably soluble in only a few, very hydrophilic, substantially non-aqueous organic solvents such as pyridine, dimethylformamide morpholine, N-methylpyrolidone and dimethylsulfoxide. Care should be taken, however, in selecting an appropriate organic solvent in that the organic solvent should be screened to assure that it does not significantly detract from the catalytic activity of the hydrolytic enzyme. Of the previously mentioned organic solvents, the solvent is selected such that the sugar and acylating compound are soluble (ie, at least about 10 mmol/liter and preferably about 100 mmol/liter) and the hydrolytic enzyme is active. Suitable solvents include pyridine and dimethylformamide. Pyridine is the preferred solvent because it solubilises a broader range of sugars than other solvents tested to date without substantially detracting from the activities of various hydrolytic enzymes.

Several hydrolytic enzymes have been found to retain their catalytic activity in either pyridine or dimethylformamide. Applicants have ascertained that the following hydrolytic enzymes are catalytically active in pyridine: Aminoacylase; Lipozyme, available from NOVO CHEMICAL; Fungal Amylase, available under the trade name "HT" from MILES KALI-CHEMIE; Bacterial protease, available under the trade name "Bioenzyme" from GIST-BROCADES; Amylase from Bacillus subtilis available under the trade name "Rapidase" from GIST-BROCADES; Alkaline protease, available under the trade name "Proleather" from AMANO; Bacillus protease available under the trade name "Protease N" from AMANO; Lipase from Candida cylindracea, available from SIGMA; Lipase from porcine pancreas, available from SIGMA; and Lipase from Penicillium Sp., available under the trade name "Lipase G" from AMANO.

The amount of hydrolytic enzyme provided to catalyze the regioselective diacylation of the sugar molecules is not critical, provided there is sufficient enzyme to initiate the diacylation of the sugars (about 10 mg/ml). By varying the amount of enzyme employed, however, the speed of the acylation can be affected. In general, increasing the amount of hydrolytic enzyme increases the speed at which the sugar is acylated. The hydrolytic enzymes useful for the acylation of the sugar are alkaline protease, amino acylase, fungal amylase, bacterial protease and subtilisin. Additionally, lipase P-30 from Pseudomonas Sp. available from AMANO has been found suitable. Preferably activated alkaline protease will be used.

Preferably, the sugar is acylated in the presence of a compound selected to inhibit the premature polymerizatin of the acylating compound. Suitable inhibitors include ascorbate and hydroquionone. Hydroquionone is preferred. Of course, other inhibitors known by those skilled in the art may be used.

The sugar and acylating compound are mixed in the presence of hydrolytic enzyme and inhibitor in the solvent at a temperature of about 10°C to about 60°C for a time sufficient to permit the acylation of the sugars (preferably about 24 hours). The sugar and acylating compound are mixed in at least a 1:1 molar ratio, with an excess of acylating compound being preferred. Once formed, the acylated sugars may be separated pursuant to silica gel chromatography or any other method of separation known by those skilled in the art.

The resulting acylated sugars have the general formula: 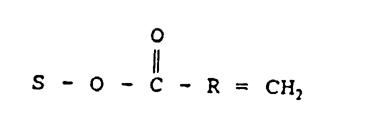 wherein S comprise sugar, and R is selected from the group consisting of alkanes, alkenes, branched alkanes, substituted alkenes, aromatic moieties, substituted aliphatic moieties, substituted aromatic moieties and mixtures thereof. With reference to the above formula, the sugars are acylated at primary hydroxyl positions.
For example, sucrose is acylated at the 1'- position, raffinose at the 1"- position, fructose at the 1-position, trehalose at the 6- position, α- and β-alkylglucosides at the 6- position, α- and β-haloglucosides at the 6- position, α- and β-alkylgalactosides at the 6- position, α- and β-halogalactosides at the 6- position, α- or β-alkylmannosides at the 6- position, and α- and β-halomannosides at the 6- position.

The acylated sugars are then polymerized by dissolving the sugars in either water or a non-aqueous organic solvent such as dimethylformamide, N-methylpyrolidone, dimethylsulfoxide or dimethyl acetamide (dimethylformamide being preferred) and thereafter sparging the resulting mixture with nitrogen for about ten minutes at about 40°C. Other methods of agitation may, of course, be used in place of sparging with nitrogen. Thereafter, a free radical initiator is added to the solution in an amount from about 0.05% to about 0.5% by weight initiator per weight acylated sugar monomers. The molecular weight of the final product is inversely proportional to the amount of initiator added.

Where water is the solvent, an equal amount of potassium persulfate and hydrogen peroxide initiators are preferably added. Where a substantially non-aqueous organic solvent is used, preferred initiators include azobisisobutyrol-nitrile, benzoyl peroxide and tert-butyl peroxide. In either case, the resulting mixture is mixed (250 rpm) at about 40°C for about 24 hours. The resulting sugar-based polymer can then be recovered by precipitation with acetone, filtered and dried.

The resulting sugar-based polymer will have the general formula: 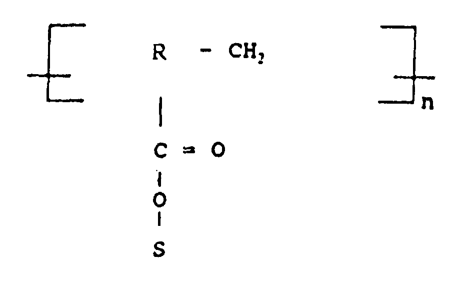 wherein S comprises non-reducing sugar, R is selected from the group consisting of alkanes, alkenes, branched alkanes, substituted alkenes, aromatic moieties, substituted aliphatic moieties, substituted aromatic moieties and mixtures thereof, and n is a whole number greater than 1. With reference to the above formula, the sugars are attached at primary hydroxyl positions. For example, sucrose is attached at the 1'-position, raffinose at the 1"-position, fructose at the 1'-position, trehalose at the 6-position, alkyl-and haloglucosides at the 4-position, alkyl-and halogalactosides at the 6-position and alkyl-and halomannosides at the 6-position.

### CROSS-LINKING OF THE SUGAR-BASED POLYMERS

In some applications it may be desirable to cross-link the sugar-based polymers of the present invention. For example, where shorter linking groups (i.e. for example the R₂ group in the previously discussed organic acid derivative) are employed (i.e. less than about 10 carbons), the polymer will be hydrophilic and potentially water soluble. Light cross-linking would result in an insoluble hydrophilic polymer that could swell and absorb water. This will be particularly important with lower molecular weight polymers. One approach of providing cross-linking capability to the sugar-based polymer is via the incorporation of an unsaturated fatty acid into the sugar-based polymer. This could be accomplished, for example, by the use of an unsaturated fatty acid in the organic diacid derivative or in the coreactant. This would result in the incorporation of unsaturated fatty acid chains in the sugar-based polymer. Heating or irradiating the polymer would cause cross-linking to occur at the unsaturated bonds resulting in a thermosetting or photosetting sugar-based polymer.

Another approach is to cross-link open hydroxyl positions on the sugars using a cross-linking species such as a diisocyanate or dinitrile. Preferred cross-linking species include 1,4-tetramethylene diisocyanate, 1,6-hexamethylene diisocyanate and 1,6-hexamethylene dinitrile in the presence of a trialkylamine catalyst (with respect to the use of a dinitrile cross-linking agent, the cross-linking is acid catalyzed rather than trialkyl amine catalyzed). For all practical purpose, however, only a limited amount of cross-linking is suggested pursuant to this method. Generally, no more than one hydroxyl per sugar moiety should be cross-linked. Excessive cross-linking may result in the removal of too many free hydroxyl groups, thereby reducing the water-absorbency of the sugar-based polymer. The polymers could also be cross-linked during the acylation process by using organic acid derivates having three or four carboxyl groups, the free carboxyl groups acting as cross-linking points. Of course, any method of cross-linking known by those skilled in the art is contemplated for use in the present invention.

It is to be understood that an equivalent of changes and modification of the above described embodiments are also contemplated for use in the present invention. The following examples are not to be construed as limitations upon the present invention, the scope of which is defined by the claims appended hereto, but are included merely as an illustration of various embodiments.

### EXAMPLES

### Example 1

In order to identify enzymes capable of catalyzing the regioselective diacylation of sucrose and, ultimately, the synthesis of sucrose-based polymers, a variety of hydrolytic enzymes were screened for their ability to synthesize sucrose butyrate in pyridine. In this manner, simple esters of sucrose were obtained and structurally analyzed without the added complication of polymer formation. Trifluoroethylbutyrate was chosen as the butyrate donor. In all, 15 enzymes were studied for sucrose-butyrate synthesis (Table 1). A typical reaction mixture contained 0.1 M sucrose dissolved in 2 mL anhydrous pyridine containing 0.6 M trifluoroethylbutyrate. The 6:1 molar ratio of trifluoroethylbutyrate to sucrose was chosen to expedite the reaction. The reactions were initiated by the addition of 0.25 g/mL enzyme (0.015 g/mL in the case of "proleather", an alkaline protease obtained from Amano) and mixing at 250 rpm and 45°C. Sucrose disappearance was monitored by HPLC. As can be discerned from Table 1, the five most active enzymes were Alkaline Protease; Bacterial Protease; Bacillus protease; Aminoacylase; and subtilisin.

### Example 2

The five most catalytically active enzymes from Example 1 were subjected to a 25 mL reaction scale (same concentrations of reactants and enzyme as in Example 1). After the time scale indicated in Table 2 the reactions were terminated and the solvent evaporated. The residual solids were chromatographed on silica gel (17:2:1; ethyl acetate:methanol:water) and the sucrose ester products separated. Clearly, as can be discerned from Table 2, the alkaline protease ("proleather") produced the highest ratio of sucrose dibutyrate to monobutyrate. The production of the sucrose dibutyrate is important for the subsequent synthesis of the sucrose-based polymer of the present invention. ¹³C-NMR analysis of the proleather mono- and diester products indicated that the sucrose is first acylated in the 1' position followed by acylation at the 6 position.

### Example 3

A poly (sucrose acrylate) was made by dissolving 3.42 g (0.1 M) sucrose in 100 ml pyridine containing 5.88 g (0.6 M) vinyl acrylate. Hydroquinone (0.5% w/v) was added inhibit polymerization of the vinyl acrylate during the sucrose acrylate synthesis. The sucrose acrylate synthesis was initiated by addition of 15 mg/ml Proleather and the mixture was magnetically stirred under nitrogen at 150 rpm for 5 days at 45°C. The reaction was terminated by filtering of the enzyme, evaporating the pyridene and unreacted vinyl acrylate, and the product was purified and separated by silica gel chromatography with an eluent consisting of ethyl acetate:methanol:water (18:1.25:1). The sucrose monoester was obtained in 28% yield, 1.10 g. The ester was an amorphous solid, mp = 78°C; [α]_{D}²⁵ = 50.4 (cl,H₂O).

Subsequent poly (sucrose acrylate) synthesis was carried out by dissolving 0.1 g (0.25 M) of the sucrose monoester in 1 ml H₂O and the solution was sparged with N₂ for ten minutes. Potassium persulfate (0.15% and 0.2% hydrogen peroxide were added and the solution was stirred at 25°C for 24 hours. The resulting poly (sucrose 1'-acrylate) was recovered by precipitation with acetone, filtered and dried under vacuum at 45°C. The poly (sucrose 1'-acrylate) was obtained in 80% yield (80 mg), and was characterized as an amorphous solid, [a]_{D} ²⁵ = 38.3 (0.67, H₂O), M_{D} = 57,000, M_{w} = 91,000. Anal. calcd. for C₁₅H₂₃O₁₂ (per repeat unit); C,45,5;H,6.1; 0,48.5; found c,43.2; H,5.9; 0,47.0. The poly (sucrose l'acrylate) was soluble in a variety of polar organic solvents including water, dimethylformamide, and N-methylpyrodlidone. As confirmed by IR analysis, the poly (sucrose 1'-acrylate) had units of the following structure:

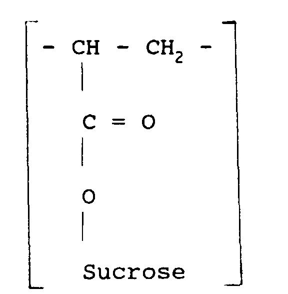

It is to be understood that a variety of sugars, organic acid derivatives, organic solvents, and hydrolytic enzymes can be substituted for those specified above and mixed in similar proportions to make various sugar-based polymers. The preceding examples should in no way be construed as limiting the extent of the present invention, the scope of which is defined by the following claims.

**TABLE 1**

| Screen of Enzymes for Sucrose-Butyrate Synthesis^{a} | |
|---|---|
| Enzyme | Sucrose Conversion (120 h) |
| Control (no enzyme) | 0% |
| Lipase from As Spergillus Sp. | 0% |
| Aminoacylase | 70% |
| Lipozyme (Novo) | 8% |
| Fungal Amylase (HT from Rohm) | 34% |
| Bacterial protease (Bioenzyme) | 100% |
| Amylase from B. subtilis (Rapidase from Gist-Brocades) | 24% |
| Rhizopus Sp. Lipase | 0% |
| Alkaline protease (Amano-Proleather) | 96% |
| Bacillus protease | 65% |
| Lipase from Pseudomonas Sp. (Amano P) | 0% |
| Lipase from C. cylindracea (Sigma) | 7% |
| Lipase from porcine pancreas (Sigma) | 13% |
| Yeast Esterase (Sturge, Ltd.) | 0% |
| Crude subtilisin (Amano protease N) | 83% (in dimethylformamide) |
| Lipase from Penicillium Sp. (Amano G) | 24% |

| | |
|---|---|
| ^{a}Conditions: Sucrose (0.1 M) dissolved in 2 Ml pyridine containing 0.6 M trifluoroethylbutyrate. Reaction initiated by addition of 0.25 g/ml enzyme and shaken at 250 rpm at 45°C. | |

**TABLE 2**

| Enzymatic Synthesis of Sucrose Butyrates^{a} | | | | |
|---|---|---|---|---|
| Enzyme | Conversion | Isolated Yield | 1'-Ester | 6.1'-Diester |
| Alkaline Protease (Proleather) | 99% (8 days) | 0.5 g (43%) | 0.12 g | 0.38 g |
| Bacterial Protease (Bioenzyme) | 100% (8 days) | 0.57 g (52%) | 0.30 g | 0.27 g |
| Bacillus Protease | 62% (21 days) | 0.39 g (37%) | 0.31 g | 0.08 g |
| Aminoacylase | 67% (23 days) | 0.54 g (49%) | 0.26 g | 0.28 g |
| Crude Subtilisin in Dimethylformamide | 62% (25 days) | 0.91 g (84%) | 0.66 g | 0.25 g |

| | | | | |
|---|---|---|---|---|
| ^{a}Conditions: Sucrose (0.1 M) dissolved in 25 Ml pyridine (except with subtilisin) containing 0.25 g/Ml enzyme and 0.6 M trifluoroethylbutyrate, magnetically stirred at 150 rpm at 45°C. | | | | |

## Claims

1. A chemoenzymatic method for making a sugar based polymer comprising units of the formula: 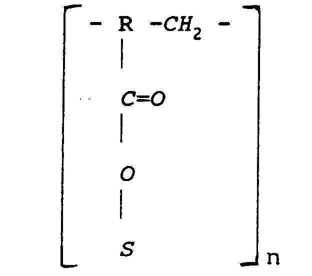 wherein S is a non-reducing sugar, R is selected from the group consisting of alkanes, alkenes, branched alkanes, substituted alkenes, aromatic moieties, substituted aliphatic moieties, substituted aromatic moieties and mixtures thereof, and n is a whole number greater than 1, which method comprises the steps of;
(a) in the presence of a hydrolytic enzyme reacting an acylating compound with a non-reducing sugar in a substantially non aqueous organic solvent to form the ester of the sugar; the acylating compound having the general formula: 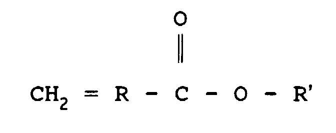 wherein R is selected from the group consisting of alkanes, alkenes, branched alkanes, substituted alkenes, substituted aliphatic moieties, aromatic moieties, substituted aromatic moieties and mixtures thereof, and R' is a moiety that is replaced by the non-reducing sugar,
(b) polymerising the ester of the sugar to form the sugar based polymer.

2. A method as claimed in claim 1 wherein the non-reducing sugar is selected from α- or β-alkyl- or α- or β-halo-glucosides, α- or β-alkyl- α- or β-halogalactosides, α- or β-alkyl- or α- or β- halomannosides, fructose, mannose, trehalose and raffinose.

3. A method as claimed in claim 1 or claim 2 wherein the sugar is selected from α- or β-methyl glucosides and α- or β-methyl galactosides.

4. A method as claimed in any one of the preceding claims wherein the acylating compound comprises vinyl acrylate.

5. A method as claimed in any one of the preceding claims wherein the hydrolytic enzyme is selected from alkaline protease, aminoacylase, fungal amylase, bacterial protease and subtilisin.

6. A method as claimed in claim 1 wherein said non-reducing sugar is sucrose.

7. A sugar based polymer with the structure: 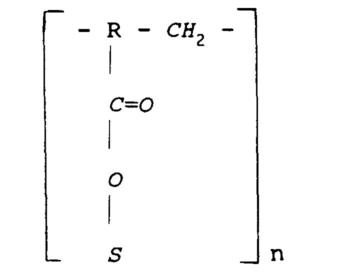 wherein S is a non-reducing sugar, R is selected from the group consisting of alkanes, alkenes, branched alkanes, substituted alkenes, aromatic moieties, substituted aliphatic moieties, substituted aromatic moieties and mixtures thereof, and n is a whole number greater than 1.

8. A sugar based polymer as claimed in claim 7 wherein the non-reducing sugar is selected from α- or β-alkyl- or α- or β-halo-glucosides, α- or β-alkyl- or α- or β-halo-galactosides, α- or β-alkyl- or α- or β-halo-mannosides, fructose, mannose, trehalose and raffinose.

9. A sugar based polymer as claimed in claim 7 wherein the sugar is selected from α- or β-alkyl- or α- or β-halo glucosides linked at the 6-position, α- or β-alkyl- or α- or β-halo-galactosides linked at the 6-position, α- or β-alkyl- or α- or β-halo-mannosides linked at the 6-position.

10. A sugar based polymer as claimed in claim 9 wherein the sugar is selected from α- or β-methyl glucosides and α- or β-methyl galactosides.

11. A sugar based polymer as claimed in claim 7 wherein said non-reducing sugar is sucrose.

## Patentansprüche

1. Chemoenzymatisches Verfahren zum Herstellen eines Polymers auf Zuckerbasis umfassend Einheiten der Formel: 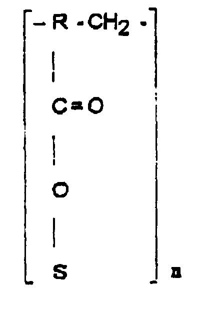 worin S ein nichtreduzierender Zucker ist, R ausgewählt ist aus der Gruppe bestehend aus Alkanen, Alkenen, verzweigten Alkanen, substituierten Alkenen, aromatischen Bestandteilen, substituierten aliphatischen Bestandteilen, substituierten aromatischen Bestandteilen und Gemischen davon, und n eine ganze Zahl größer als 1 ist, wobei das Verfahren die Schritte umfaßt:
(a) Umsetzen einer acylierenden Verbindung mit einem nichtreduzierenden Zucker in einem im wesentlichen nichtwäßrigen organischen Lösungsmittel in Gegenwart einer Hydrolase, um den Ester des Zuckers zu bilden; wobei die acylierende Verbindung die allgemeine Formel aufweist: 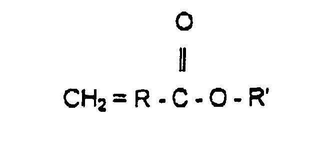 worin R ausgewählt ist aus der Gruppe bestehend aus Alkanen, Alkenen, verzweighten Alkanen, substituierten Alkenen, substituierten aliphatischen Bestandteilen, aromatischen Bestandteilen, substituierten aromatischen Bestandteilen und Gemischen davon, und R' ein Bestandteil ist, der durch den nichtreduzierende Zucker ersetzt wird,
(b) Polymerisieren des Esters des Zuckers, um das Polymer auf Zuckerbasis zu bilden.

2. Verfahren wie in Anspruch 1 beansprucht, worin der nichtreduzierende Zucker ausgewählt ist aus α-oder β-Alkyl- oder α-oder β-Halogen-Glucosiden, α-oder β-Alkyl-oder α-oder β-Halogengalactosiden, α-oder β-Alkyl- oder α-oder β-Halogen-mannosiden, Fructose, Mannose, Trehalose und Raffinose.

3. Verfahren wie in Anspruch 1 oder Anspruch 2 beansprucht, worin der Zucker aus-gewählt ist aus α-oder β-Methylglucosiden und α-oder β-Methylgalactosiden.

4. Verfahren wie in einem der vorangehenden Ansprüche beansprucht, worin die acylierende Verbindung Vinylacrylat umfaßt.

5. Verfahren wie in einem der vorangehenden Ansprüche beansprucht, worin die Hydrolase ausgewählt ist aus alkalischer Protease, Aminoacylase, pilzlicher Amylase, bakterieller Protease und Subtilisin.

6. Verfahren wie in Anspruch 1 beansprucht, worin der nichtreduzierende Zucker sucrose ist.

7. Polymer auf Zuckerbasis mit der Struktur: 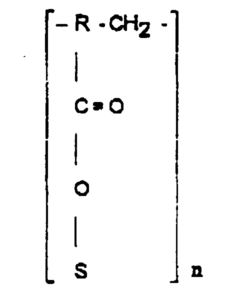 worin S ein nichtreduzierende Zucker ist, R ausgewählt ist aus der Gruppe bestehend aus Alkanen, Alkenen, verzweighten Alkanen, substituierten Alkenen, aromatischen Bestandteilen, substituierten aliphatischen Bestandteilen, substituierten aromatischen Bestandteilen und Gemischen davon, und n eine ganze Zahl größer als 1 ist.

8. Polymer auf Zuckerbasis wie in Anspruch 7 beansprucht, worin der nichtreduzierende Zucker ausgewählt ist aus α-oder β-Alkyl- oder α-oder β-Halogen-Glucosiden, α-oder β-Alkyl- oder α-oder β-Halogengalactosiden, α- oder β-Alkyl-oder α-oder β-Halogenmannosiden, Fructose, Mannose, Trehalose und Raffinose.

9. Polymer auf Zuckerbasis wie in Anspruch 7 beansprucht, worin der Zucker ausgewählt ist aus α-oder β-Alkyl- oder α-oder β-Halogenglucosiden, die in 6-Stellung verknüpft sind, α-oder β-Alkyl- oder α-oder β-Halogengalactosiden, die in 6-Stellung verknüpft sind, α-oder β-Alkyl- oder α-oder β-Halogenmannosiden, die in 6-Stellung verknüpft sind.

10. Polymer auf Zuckerbasis wie in Anspruch 9 beansprucht, worin der Zucker ausgewählt ist aus α-oder β-Methylglucosiden und α-oder β-Methylgalactosiden.

11. Polymer auf Zuckerbasis wie in Anspruch 7 beansprucht, worin der nichtreduzieriende Zucker sucrose ist.

## Revendications

1. Procédé chimio-enzymatique de production d'un polymère à base de sucre comprenant des motifs de formule : 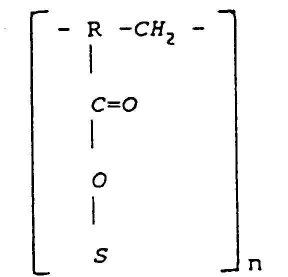 dans laquelle S est un sucre non réducteur, R est choisi dans le groupe consistant en alcanes, alcènes, alcanes ramifiés, alcènes substitués, fragments aromatiques, fragments aliphatiques substitués, fragments aromatiques substitués et leurs mélanges, et n est un nombre entier supérieur à 1, procédé qui comprend les étapes de :
(a) réaction, en présence d'un enzyme hydrolytique, d'un composé acylant avec un sucre non réducteur dans un solvant organique sensiblement non aqueux pour former l'ester du sucre ; le composé acylant répondant à la formule générale : 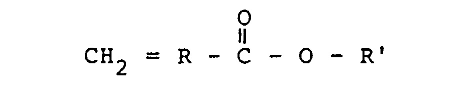 dans laquelle R est choisi dans le groupe consistant en alcanes, alcènes, alcanes ramifiés, alcènes substitués, fragments aliphatiques substitués, fragments aromatiques, fragments aromatiques substitués et leurs mélanges, et R' est un fragment qui est remplacé par le sucre non réducteur,
(b) polymérisation de l'ester du sucre pour former le polymère à base de sucre.

2. Procédé suivant la revendication 1, dans lequel le sucre non réducteur est choisi entre des α-ou β-alkyl- ou des α- ou β-halogéno-glucosides, des α- ou β-alkyl- ou des α- ou β-halogéno-galactosides, des α- ou β-alkyl- ou des α- ou β-halogéno-mannosides, le fructose, le mannose, le tréhalose et le raffinose.

3. Procédé suivant la revendication 1 ou la revendication 2, dans lequel le sucre est choisi entre des α ou β-méthyl-glucosides et des α- ou β-méthyl-galactosides.

4. Procédé suivant l'une quelconque des revendications précédentes, dans lequel le composé acylant comprend l'acétate de vinyle.

5. Procédé suivant l'une quelconque des revendications précédentes, dans lequel l'enzyme hydrolytique est choisi entre une protéase alcaline, une aminoacylase, une amylase fongique, une protéase bactérienne et la subtilisine.

6. Procédé suivant la revendication 1, dans lequel le sucre non reducteur est le sucrose.

7. Polymère à base de sucre ayant la structure: 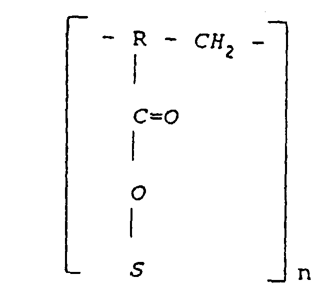 dans laquelle S est un sucre non réducteur, R est choisi dans le groupe consistant en alcanes, alcènes, alcanes ramifiés, alcènes substitués, fragments aromatiques, fragments aliphatiques substitutés, fragments aromatiques substitués et leur mélanges, et n est un nombre entier supérieur à 1.

8. Polymère à base de sucre suivant la revendication 7, dans lequel le sucre non réducteur est choisi entre des α- ou β-alkyl- ou des α- ou β-halogénoglucosides, des α- ou β-alkyl- ou des α- ou β-halogénogalactosides, des α- ou β-alkyl- ou des α- ou β-halogénomannosides, le fructose, le mannose, le tréhalose, le et le raffinose.

9. Polymère à base de sucre suivant la revendication 7, dans lequel le sucre est choisi entre des α- ou β-alkyl- ou des α- ou β-halogéno-glucosides liés en position 6, des α- ou β-alkyl- ou des α- ou β-halogénogalactosides liés en position 6, des α- ou β-alkyl- ou des α- ou β-halogéno-mannosides liés en position 6.

10. Polymère à base de sucre suivant la revendication 9, dans lequel le sucre est choisi entre des α- ou β-méthyl-glucosides et des α- ou β-méthylgalactosides.

11. Polymère à base de sucre suivant la revendication 7, dans lequel le sucre non-reducteur est le sucrose.
